# EUROPEAN PATENT APPLICATION

(11) **EP 1 398 006 A2**
(43) Date of publication of application: **17.03.2004**
(21) Application number: 03255591.4
(22) Date of filing: 09.09.2003
(51) Int. Cl.: A61F 2/30, A61F 2/38

(54) **Duo-fixation prosthetic joints**

(30) Priority: 09.09.2002 US 409262 P; 15.08.2003 US 642088
(71) Applicant: Depuy Products, Inc., Warsaw, Indiana 46581 (US)
(72) Inventor: Haas, Brian, Engelwood, Colorada (US)
(74) Representative: Belcher, Simon James

(57) **Abstract**

An orthopaedic implant system has an orthopaedic component (12) with a stem (22) for receipt in a bone. The stem has a rough outer surface to provide a bonding surface for cement or to provide a substrate for bony ingrowth for fixing the implant in the bone. The system also includes a sleeve (16) for use with the stem of the orthopaedic component. The surgeon can opt to use the orthopaedic component alone without any sleeve or can use the orthopaedic component with a sleeve if the patient's condition warrants.

## Description

The present invention relates generally to prosthetic joints and, more particularly, to prosthetic knee joints. The prosthetic joint components of the present invention can be fixed to the patient's bone by different methods.

The knee joint basically consists of the bone interface of the distal end of the femur and the proximal end of the tibia. Appearing to cover or at least partially protect this interface is the patella which is a sesamoid bone within the tendon of the long muscle (quadriceps) on the front of the thigh. This tendon inserts into the tibial tuberosity and the posterior surface of the patella is smooth and glides over the femur.

The femur is configured with two knob like processes (the medial condyle and the lateral condyle) which are substantially smooth and articulate with the medial plateau and the lateral plateau of the tibia, respectively. The plateaus of the tibia are substantially smooth and slightly cupped thereby providing a slight receptacle for receipt of the femoral condyles.

When the knee joint is injured whether as a result of an accident or illness, a prosthetic replacement of the damaged joint may be necessary to relieve pain and to restore normal use to the joint. Typically the entire knee joint is replaced by means of a surgical procedure which involves removal of the ends of the corresponding damaged bones and replacement of these ends with prosthetic implants. This replacement of a native joint with a prosthetic joint is referred to as a primary total-knee arthroplasty.

On occasion, the primary knee prostheses fails. Failure can result from many causes, including wear, aseptic loosening, osteolysis, ligamentous instability, arthrofibrosis and patellofemoral complications. When the failure is debilitating, revision knee surgery may be necessary. In a revision, the primary knee prosthesis is removed and replaced with components of a revision prosthetic knee system.

In knee surgery or arthroplasty, the prosthetic components can typically be fixed to the patient's bone in different ways. In one type of prosthetic joint system, the components may include a sleeve that initially has an interference fit with the patient's bone, and in which bony ingrowth helps to secure the implant and the bone together. Components for this type of fixation typically have a series of stepped outer surfaces, tapering from one end to an opposite end, as disclosed in United States Pat. Nos. 4,790,852 and 4,846,839, the complete disclosures of which are incorporated by reference herein. In these systems, the stepped outer surfaces are provided on a sleeve that fits over a stem of a separate prosthetic component. The stem of these separate components has a smooth outer surface, and the inner surface of the sleeve that slides over this outer stem surface is also smooth. Generally, the outer surface of the stem is highly polished, with a typical surface roughness of 4 Rₐ or better as determined by ANSI/ASME B46.1-1995. The outer surface of the sleeve is typically rough for use in both cemented fixation or to allow for bony ingrowth. This system has been used in both prosthetic hip and knee joints.

Another type of prosthetic system does not use such tapered, stepped sleeves. Such systems typically have components with stems that fit into a prepared cavity or the intramedullary canal of the patient's bone. The stems and other surfaces of the prosthesis that may contact bone are typically rough, either for fixation of the surfaces to bone cement that holds the implant to the bone or for bony ingrowth.

Due in part to the fact that the size, shape and anatomy of virtually every patient is different, an extensive number of a variety of components have been made available to the orthopaedic surgeon in the typical surgical kit. For example, a typical surgical kit for knee arthroplasty will include femoral and tibial components of different configurations to accommodate variations in patient anatomy. In addition, if the surgeon desired to chose between different types of prosthetics intraoperatively, it was necessary for complete surgical sets of the different types of systems to be available to the surgeon. To provide the surgeon with these alternatives would require that a large inventory be on hand and available to the surgeon.

The present invention addresses the need to provide the surgeon with greater options to select the optimal prosthetic joint intraoperatively.

In one aspect, the present invention addresses this need by providing a prosthetic implant surgical kit for use in joint arthroplasty. The kit comprises a plurality of orthopaedic implants and a plurality of sleeves. Each orthopaedic implant has a stem for receipt in a bone. All stems of all the orthopedic implants have a rough outer surface. The sleeves each have outer surfaces and inner channels for mounting the sleeves on the stems of the implants.

In another aspect, the present invention addresses this need by providing an orthopaedic implant assembly comprising an orthopaedic component having a stem with a distal end, a proximal end and a rough outer surface between the distal end and the proximal end. The assembly also includes a sleeve mounted on the stem of the orthopaedic component. The sleeve has a smooth surface juxtaposed with the rough outer surface of the stem.

In another aspect, the present invention addresses this need by providing an orthopaedic assembly comprising a sleeve having an outer surface and an inner surface. The inner surface of the sleeve defines a smooth frusto-conical channel. The assembly also includes an orthopaedic component with a stem received in the smooth channel of the sleeve. The stem of the orthopaedic component has a rough outer surface.

Embodiments of the invention will now be described by way of example with reference to the accompanying drawings, in which:
FIG. 1 is perspective view of a tibial component of a prosthetic knee system incorporating the principles of the present invention;
FIG. 2 is a front elevation of a tibial sleeve to be used with the tibial component of FIG. 1;
FIG. 3 is a side elevation of the tibial sleeve of FIG. 2;
FIG. 4 is a top plan view of the tibial sleeve of FIGS. 2-3;
FIG. 5 is a cross-section of the tibial sleeve of FIGS. 2-4, taken along line taken along line 5-5 of FIG. 3;
FIG. 6 is a front elevation of an assembly of the tibial component of FIG. 1 with the tibial sleeve of FIGS. 2-5;
FIG. 7 is a cross-section of the tibial sleeve of FIG. 6, showing the tibial stem of the tibial component in full and received within the tibial sleeve;
FIG. 8 is a side elevation of a femoral component to be used with the tibial component of FIG. 1 or with the assembly of FIG. 6;
FIG. 9 is a perspective view of a femoral sleeve for optional use with the femoral component of FIG. 8 with an end cap at its proximal end;
FIG. 10 is a side elevation of the femoral sleeve of FIG. 9;
FIG. 11 is a cross-section of the femoral sleeve of FIGS. 9-10, taken along line 11-11 of FIG. 10;
FIG. 12 is a side elevation of an assembly of the femoral component of FIG. 8 with the femoral sleeve of FIGS. 9-10 for use with the tibial component of FIG. 1 or with the tibial assembly of FIGS. 6-7; and
FIG. 13 is a cross-section of the femoral sleeve of FIG. 11, showing the femoral stem of the femoral component in full and received within the femoral sleeve.

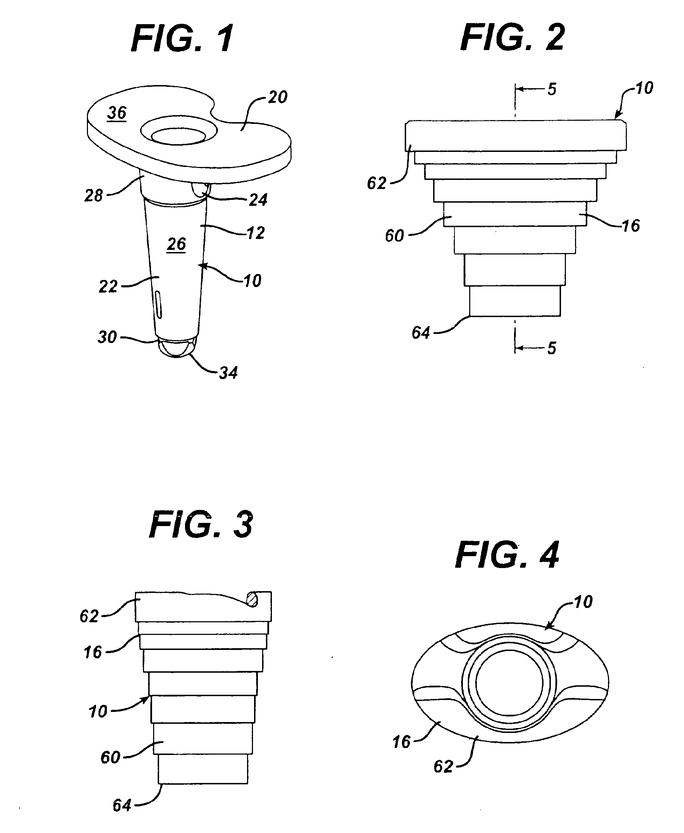

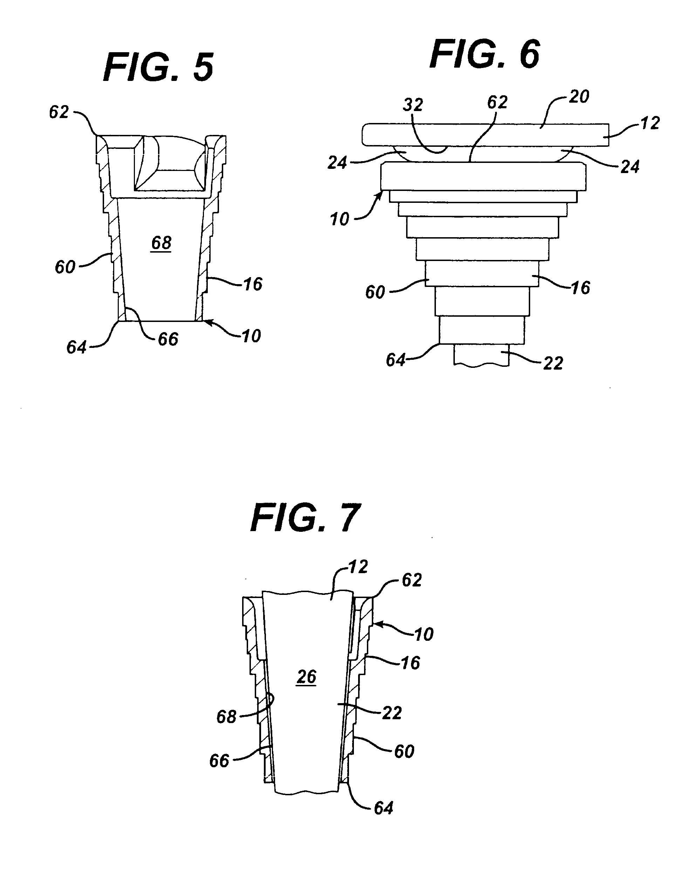

Referring to the drawings, FIGS. 1 to 12 show a modular prosthetic joint system 10 which includes a tibial component 12, a femoral component 14, a tibial bearing insert (not shown), a tibial sleeve 16 and a femoral sleeve 18. It should be understood that a typical surgical kit for the prosthetic joint system 10 will probably include multiple sizes of each of these components so that the surgeon can select the most appropriate size components for each patient.

As shown in FIG.1, the tibial component 12 comprises a tibial tray 20 with an integral stem 22 and integral keels 24. The tibial stem 22 in the illustrated embodiment has a rough outer surface 26 from its proximal end 28 at the juncture with the tibial tray 20 to its distal end 30. The distal surface 32 of the tibial tray 20 fits against the proximal surface of the resected tibia or against an augment when implanted, and may also have a rough surface. The tibial stem 22 is received in the cancellous bone of the proximal tibia, and extends into the intramedullary canal of the tibia. The distal end 30 of the tibial stem may be connected to a stem extension (not shown) that is received in the tibial intramedullary canal or to an end cap 34 shown in FIG. 1. The tibial stem 22 may be hollow to receive a portion of the tibial insert bearing, and the proximal surface 36 of the tibial tray 20 supports the insert bearing. The proximal surface 36 of the tibial tray 20 is smooth.

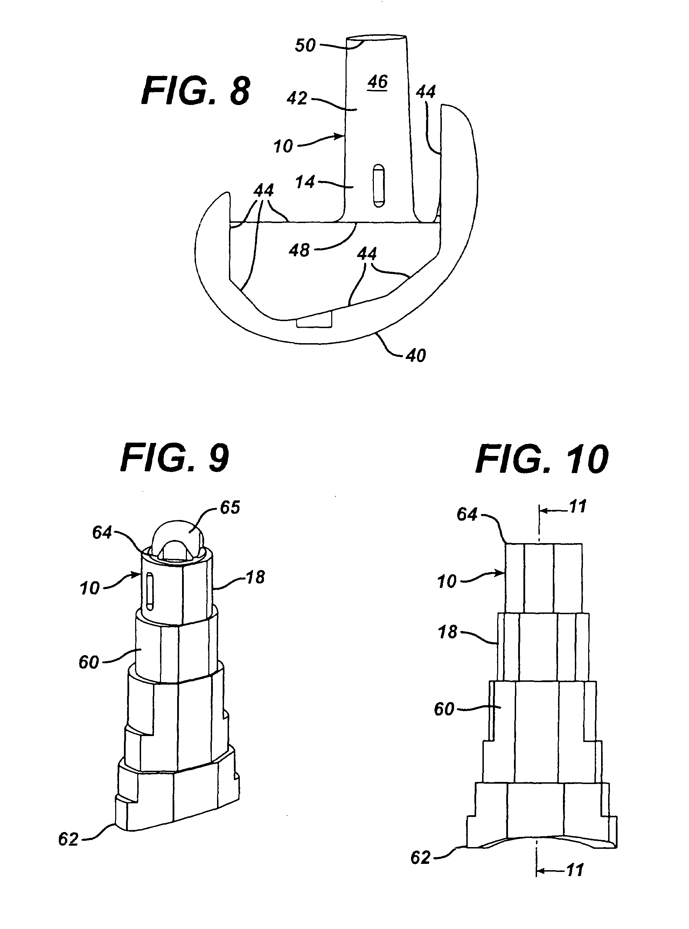

The femoral component 14 includes outer condylar portions 40 that bear against the tibial insert bearing in use. As shown in FIG. 8, the femoral component 14 also includes an integral femoral stem 42 and inner surfaces 44 that will bear against a resected surface of the femur or against an augment (not shown) that is positioned between the femoral component and the resected bone surface. The femoral stem 42 is received in the cancellous bone of the distal femur, and extends into the intramedullary canal of the femur. The femoral stem 42 in the illustrated embodiment has a rough outer surface 46 from its distal end 48 at the juncture with the flat inner surfaces of the femoral component to its proximal end 50. The proximal end 50 of the femoral stem may be connected to a stem extension (not shown) to be received in the femoral intramedullary canal.

The tibial bearing insert can be a standard commercially available insert, such as is available from DePuy Orthopaedics, Inc. of Warsaw, Indiana.

The tibial sleeve 16 and femoral sleeve 18 are commercially available from DePuy Orthopaedics of Warsaw, Indiana. They each have stepped or terraced outer surfaces 60 that taper from one end 62 to an opposite end 64. Each sleeve 16, 18 has an inner channel 66 defined by a smooth inner surface 68. The femoral sleeve 18 is designed to be placed over the femoral stem 42 so that the assembly of the femoral component 14 and femoral sleeve 18 can be implanted in the patient when desired. The tibial sleeve 16 is similarly designed to be placed over the tibial stem 22 so that the assembly of the tibial component 12 and tibial sleeve 16 can be implanted in the patient when desired. The femoral sleeve 18 can be used with an end cap 65, as shown in FIG. 9.

The tibial component and tibial sleeve may have features as described in the European patent applications filed with this application which claim priority from US patent application no. 60/409284, entitled "Prosthetic tibial component with modular sleeve", and US patent application no. 60/409319, entitled "Universal tibial augment".

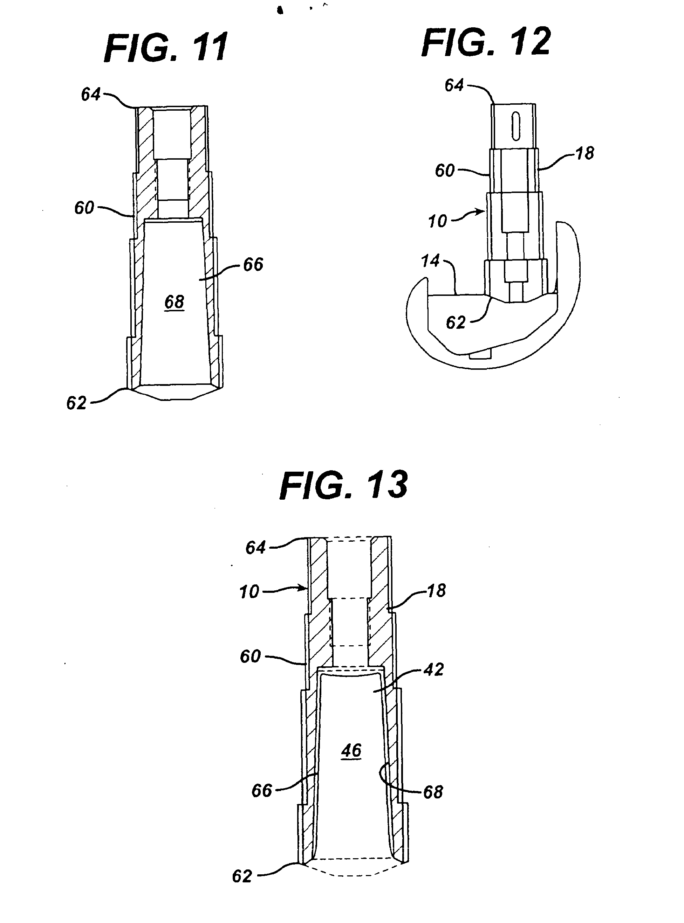

In the prior art, the juxtaposed outer surfaces of the stems and inner surfaces of the sleeves were both smooth to allow for easy assembly of the stems and sleeves. In contrast, in the present invention, the juxtaposed outer surfaces 26, 46 of the stems 22, 42 and inner surfaces 58 of the channels 66 of the sleeves 16, 18 have different finishes. In the assemblies of the present invention, the smooth inner surfaces 58 of the sleeves 16, 18 are juxtaposed with the rough outer surfaces 26, 46 of the stems 22, 42. These juxtaposed surfaces 26 and 68 and 46 and 48 are illustrated in FIGS. 7 and 13. Advantageously, the surgeon can opt intraoperatively to use the femoral component 14 and tibial component 12, or one of them, without the associated sleeve 18, 16, and the same femoral component 14 and tibial component 12 can also optionally be implanted as an assembly with a sleeve 18, 16 if the surgeon so chooses. The surgeon has these options available in a single surgical kit, without the need to maintain multiple inventories of different types of prosthetic systems.

The femoral and tibial components 14, 12 of the prosthetic joints may be made of standard materials for such implants, such as a cast cobalt-chrome or titanium alloy, and can be made in the standard way. The rough surfaces 26, 46 on the components 12, 14 can be created by blasting the cast surface with a glass bead blast to roughen these surfaces. For example, the surfaces may be blasted with glass beads to produce a roughened surface with an average roughness Rₐ of at least about 20 µm as determined by ANSI/ASME B46.1-1995. To reduce the possibility of fretting of the rough surface when a sleeve is mounted on the stem, it may be desirable to maintain the surface roughness Rₐ of the sleeve below 45 µm. Those in the art will appreciate that manufacturing parameters such as glass bead or grit size and the pressure of the blast can be varied to achieve the desired roughness. It should be understood that the roughness Rₐ of 20 to 45 µm is provided as an example only; the present invention is not limited to a particular numerical range of roughness unless expressly called for in the claims. Generally, the surface texture of the stem should allow for successful use of the implant with bone cement and should not fret when a sleeve is mounted on the stem. In addition, the invention is not limited to use of glass bead blasting to achieve this roughness. Unless expressly excluded by the claims, the term "rough" should be construed to encompass any treatment of the surface that is appropriate for cemented implantation or for bony ingrowth. The roughening technique should be one that allows the tapered stems to be received in the tapered inner channels 66 of the sleeves 16, 18.

Generally, the inner surfaces 68 of the sleeves 16, 18 defining the inner channels 66 are smooth rather than rough, and have surface roughnesses less than that of the outer surfaces 26, 46 of the stems. For example, it may be desirable that the inner surfaces 68 be highly polished (to a surface roughness of, for example 2 to 4 Rₐ) to reduce or eliminate fretting when the sleeve is used with the roughened stem.

In use, the surgeon can prepare the proximal end of the tibia and distal end of the femur in the standard manner, and resect these bones in the standard manner to receive the prosthetic components. If the surgeon determines that the patient's bone, such as the proximal tibia, is sufficiently healthy to receive and hold the implant in a stable position, the surgeon can implant the tibial component 12 alone without a sleeve 16, and the rough surface 26 of the stem 22 of the tibial component 12 will provide a surface suitable for either bonding with bone cement or for bony ingrowth to occur. If the surgeon determines that the patient's bone has deteriorated to the point that use of a tibial sleeve 16 is desirable, the surgeon can use the same tibial component 12 and place a tibial sleeve 16 on the stem 22 of the tibial component 12 and implant the assembly of modular components 12, 16. Although the rough surface 26 of the tibial stem 22 would be effective in providing a bond to the surrounding bone or to cement, by providing the system of the present invention, the surgeon's options are increased without adding to the inventory of prosthetic devices that must be maintained. The surgeon can make the same choices on the femoral side, and can opt to use either the femoral component 14 alone or as an assembly with the femoral sleeve 18, depending on the surgeon's assessment during surgery of the optimum system for that patient.

It should be understood that although the invention has been described with regard to the illustrated femoral and tibial components, the principles of the present invention can be applied to other prosthetic joint components as well, such as those used for hip arthroplasty.

## Claims

1. A prosthetic implant surgical kit for use in joint arthroplasty, the kit comprising:
a plurality of orthopaedic implants, each orthopaedic implant having a stem for receipt in a bone, wherein all stems of all the orthopedic implants have outer surfaces with a non-smooth finish;
a plurality of sleeves having outer surfaces and inner channels for mounting the sleeves on the stems of the implants.

2. The kit of claim 1 wherein the plurality of orthopaedic implants include a plurality of orthopaedic implants for implantation on the proximal end of one bone and a plurality of orthopaedic implants for implantation on the distal end of another bone.

3. The kit of claim 2 wherein the orthopaedic implants include femoral components and tibial components.

4. The kit of claim 1 wherein the sleeves have stepped outer surfaces.

5. The kit of claim 1 wherein the non- outer surfaces of the stems are roughened by blasting.

6. The kit of claim 1 wherein the stems and inner channels of the sleeves are tapered to allow for a Morse taper fit between the stems and the sleeves.

7. The kit of claim 1 wherein the outer surfaces of the stems have roughnesses Rₐ greater than 4 µm as determined by ANSI/ASME B46.1-1995.

8. The kit of claim 1 wherein the outer surfaces of the stems have roughnesses Rₐ of at least 20 µm as determined by ANSI/ASME B46.1-1995.

9. The kit of claim 1 wherein the outer surfaces of the stems have roughnesses Rₐ of 20 to 45 µm as determined by ANSI/ASME B46.1-1995.

10. An orthopaedic implant assembly comprising:
an orthopaedic component having a stem with an outer surface with a non-smooth texture;
a sleeve mounted on the stem of the orthopaedic component, the sleeve having a smooth surface juxtaposed with the rough outer surface of the stem.

11. The assembly of claim 10 wherein the sleeve substantially covers the outer surface of the stem of the orthopaedic component.

12. The assembly of claim 10 wherein the sleeve has a stepped outer surface.

13. The assembly of claim 10 wherein the orthopaedic component comprises a femoral component.

14. The assembly of claim 10 wherein the orthopaedic component comprises a tibial component.

15. The assembly of claim 10 wherein the outer surface of the stem is roughened by blasting.

16. The assembly of claim 10 wherein the outer surface of the stem has a roughness Ra greater than 4 µm as determined by ANSI/ASME B46.1-1995.

17. The assembly of claim 18 wherein the outer surface of the stem has a roughness Rₐ of at least 20 µm as determined by ANSI/ASME B46.1-1995.

18. The assembly of claim 18 wherein the outer surface of the stem has a roughness Rₐ of 20-45 µm as determined by ANSI/ASME B46.1-1995.

19. The assembly of claim 10 wherein the stem and inner channel of the sleeve are tapered to allow for a Morse taper fit between the stem and the sleeve.

20. A prosthetic implant surgical kit for use in joint arthroplasty, the kit comprising:
an orthopaedic implant having a stem for receipt in a bone; and
a sleeve having an outer surface and an inner channel for mounting the sleeve on the stem of the orthopaedic implant;
wherein the stem of the orthopaedic implant has an outer surface with sufficient roughness for use with bone cement.

21. The kit of claim 20 wherein the sleeve has a stepped outer surface.

22. The kit of claim 20 wherein the orthopaedic implant comprises a femoral component.

23. The kit of claim 20 wherein the orthopaedic implant comprises a tibial component.

24. The kit of claim 20 wherein the roughness of the outer surface of the stem is produced by blasting.

25. The kit of claim 20 wherein the outer surface of the stem has a roughness Rₐ greater than 4 µm as determined by ANSI/ASME B46.1-1995.

26. The kit of claim 20 wherein the outer surface of the stem has a roughness Rₐ of at least 20 µm as determined by ANSI/ASME B46.1-1995.

27. The kit of claim 18 wherein the outer surface of the stem has a roughness Rₐ of 20-45 µm as determined by ANSI/ASME B46.1-1995.

28. The kit of claim 20 wherein the stem and inner channel of the sleeve are tapered to allow for a Morse taper fit between the stem and the sleeve.

29. A prosthetic implant surgical kit for use in joint arthroplasty, the kit comprising:
an orthopaedic implant having a stem for receipt in a bone; and
a sleeve having an outer surface and an inner surface defining an inner channel for mounting the sleeve on the stem of the orthopaedic implant;
wherein the stem of the orthopaedic implant has an outer surface with a roughness greater than the roughness of the inner surface of the sleeve.

30. The kit of claim 29 wherein the sleeve has a stepped outer surface.

31. The kit of claim 29 wherein the orthopaedic implant comprises a femoral component.

32. The kit of claim 29 wherein the orthopaedic implant comprises a tibial component.

33. The kit of claim 29 wherein the roughness of the outer surface of the stem is produced by blasting.

34. The kit of claim 29 wherein the outer surface of the stem has a roughness Rₐ greater than 4 µm as determined by ANSI/ASME B46.1-1995.

35. The kit of claim 36 wherein the outer surface of the stem has a roughness Rₐ of at least 20 µm as determined by ANSI/ASME B46.1-1995.

36. The kit of claim 37 wherein the outer surface of the stem has a roughness Rₐ of 20 to 45 µm as determined by ANSI/ASME B46.1-1995.

37. The kit of claim 29 wherein the stem and inner channel of the sleeve are tapered to allow for a Morse taper fit between the stem and the sleeve.

38. A prosthetic implant surgical kit for use in joint arthroplasty, the kit comprising:
an orthopaedic implant having a stem for receipt in a bone; and
a sleeve having an outer surface and an inner surface defining an inner channel for mounting the sleeve on the stem of the orthopaedic implant;
wherein the stem of the orthopaedic implant has an outer surface that has been roughened by blasting.

39. The assembly of claim 38 wherein the outer surface of the stem has a roughness Rₐ greater than 4 µm as determined by ANSI/ASME B46.1-1995.

40. The kit of claim 38 wherein the outer surface of the stem has a roughness Rₐ of at least 20 µm as determined by ANSI/ASME B46.1-1995.

41. The kit of claim 39 wherein the outer surface of the stem has a surface roughness Rₐ of 20 to 45 µm as determined by ANSI/ASME B46.1-1995.

42. The kit of claim 39 wherein the stem and inner channel of the sleeve are tapered to allow for a Morse taper fit between the stem and the sleeve.
